(19) Europäisches Patentamt
European Patent Office
Office européen des brevets

(11) **EP 4 410 195 A1**

(12) **EUROPEAN PATENT APPLICATION**

(43) Date of publication:
**07.08.2024 Bulletin 2024/32**

(21) Application number: **24153141.7**

(22) Date of filing: **22.01.2024**

(51) International Patent Classification (IPC):
***A61B 5/12*** (2006.01)

(52) Cooperative Patent Classification (CPC):
**A61B 5/126; A61B 5/6817;** H04R 25/70

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **01.02.2023 EP 23154381**

(71) Applicant: **Interacoustics A/S**
**5500 Middelfart (DK)**

(72) Inventor: **NØRGAARD, Kren Monrad**
**DK-5500 Middelfart (DK)**

(74) Representative: **Demant**
**Demant A/S**
**Kongebakken 9**
**2765 Smørum (DK)**

(54) **COMPENSATION FOR THE MIDDLE-EAR IMPEDANCE MISMATCH IN OTOACOUSTIC-EMISSION MEASUREMENTS**

(57)    The present application relates to a device for measuring otoacoustic emissions (OAE) of a test subject. The device comprises a stimulus generator configured to generate at least one acoustic stimulus, an ear probe connected to said stimulus generator, where the ear probe comprises an output unit for emitting said at least one acoustic stimulus into an ear canal of the test subject, and an input unit for measuring an acoustic emission ($P_{spl}$) from the ear of the test subject, and an analysis unit connected to the ear probe, where the analysis unit is configured to receive said measured acoustic emission ($P_{spl}$) and to determine a compensated pressure level ($P_{npl}$) of said measured acoustic emission ($P_{spl}$), where the determination of the compensated pressure level is based on characterizing the middle ear of the test subject as a Norton-equivalent circuit. The present application further relates to a method of measuring otoacoustic emissions (OAE) of a test subject and to a computer program.

FIG. 1

**Description**

SUMMARY

The present application relates to a device for measuring otoacoustic emissions (OAEs) of a test subject.
The present application further relates to a method of measuring OAEs of a test subject.
The present application further relates to a computer program comprising instructions which, when the program is executed by a device, instruct the device to carry out the steps of the method.

A Device

Otoacoustic emissions (OAEs) are faint sounds generated by active mechanisms in the cochlea. The presence of OAEs reflect outer hair-cell function and has been used for screening and diagnostic purposes to assess the health of the cochlea for decades. OAEs are categorized into spontaneous and evoked OAEs, where mostly evoked OAEs are used in the clinic.
Different types of evoked OAEs are conventionally measured using an ear probe, comprising at least one speaker and a microphone, placed in the ear canal. One or more of the speakers emit acoustic stimuli, the level of which are adjusted using the microphone. The microphone further measures the OAE response from the cochlea that is emitted into the ear canal through the middle ear.
A more recent utilization of OAEs includes, e.g., monitoring for ototoxic drugs which may cause OAE responses to deteriorate overtime. However, such monitoring procedures require that OAE responses can be accurately reproduced over time, which is inhibited by variations in the manual placement of the ear probe in the ear canal and the acoustic properties of the middle ear.
In particular, standing waves between the tympanic membrane, where an OAE enters the ear canal, and the ear probe influence the OAE response, resulting in amplification of the OAE response near resonance frequencies. The result is that OAE responses vary significantly when the ear probe is not exactly placed in the same location between measurement sessions.
A solution to this problem has already been proposed and demonstrated using the so-called emitted pressure level [1]. The basic idea of the emitted pressure level is to estimate the sound pressure that would have been recorded near the tympanic membrane in an anechoic ear canal, i.e., an anechoic ear canal free from reflections. That is, the emitted pressure level is unaffected by the insertion of the ear probe and standing-wave resonances. The emitted pressure level, $P_{epl}$ is calculated as:

$$P_{epl} = P_{spl} \frac{1 - R_{ec}R_s}{T(1 + R_s)}$$

where $P_{spl}$ is the OAE response measured in the ear canal, $R_{ec}$ is the reflectance of the ear canal, $R_s$ is the source reflectance, i.e., of the ear probe, and $T = e^{-jkl}$ is the one-way propagation coefficient corresponding to the ear-canal length $l$, under the assumption of a uniform ear canal. The ear-canal reflectance $R_{ec}$ is usually measured by the same ear probe used for the OAE measurement and enabled by a preliminary Thévenin-equivalent calibration of the probe [2]. The source reflectance $R_s$ is given from the ear-probe Thévenin-equivalent calibration.
The OAE response is further altered by the conductive characteristics of the middle ear. An approach to characterize the middle ear as an acoustic Thévenin-equivalent circuit where the equivalent pressure source constitutes the OAE response has further been described [1]. In theory, this approach removes the influence of the middle ear on the OAE response. Using identical acoustical variables, the Thévenin pressure level $P_{tpl}$ is calculated as

$$P_{tpl} = P_{epl} \frac{2}{1 - R_{ec}/T^2} = P_{spl} \frac{2T(1 - R_{ec}R_s)}{(1 + R_s)(T^2 - R_{ec})}$$

This approach, however, does not work well in practice, mainly because the middle-ear impedance (i.e., the tympanic membrane) is much larger than the characteristic impedance of the ear canal. That is, when OAEs are emitted into the ear canal, the middle ear behaves more like a flow source than a pressure source. The method is therefore extremely sensitive to accurately estimating the length of the ear canal (which is encoded into 7), which is an ill-defined quantity due to the non-uniform nature of the ear canal and oblique orientation of the tympanic membrane.
Thus, there is a need for an improved approach for determining a compensated pressure level of acoustic emissions from the ear of a test subject.

In an aspect of the present application, a device for measuring OAEs of a test subject is provided.

The device may comprise a stimulus generator configured to generate at least one acoustic stimulus.

The device may comprise an ear probe connected to said stimulus generator.

The ear probe may comprise an output unit for emitting said at least one acoustic stimulus into an ear canal of the test subject.

For example, the output unit may comprise a transducer, e.g., comprising a loudspeaker (also termed a receiver), for emitting said at least one acoustic stimulus.

The ear probe may comprise an input unit for measuring an acoustic emission ($P_{spl}$) from the ear of the test subject.

Acoustic emission ($P_{spl}$) from the ear of the test subject may refer to the acoustic emission generated in the inner ear and entering the ear canal through the middle ear and the tympanic membrane.

The input unit may comprise a transducer for measuring said acoustic emission ($P_{spl}$).

The transducer may comprise or constitute at least one microphone.

Measuring an acoustic emission may refer to measuring an OAE from the cochlea that is emitted into the ear canal through the middle ear of the test subject.

The device may comprise an analysis unit connected to the ear probe.

The analysis unit may be configured to receive said measured acoustic emission ($P_{spl}$), and/or to receive a signal representing said measured acoustic emission ($P_{spl}$), and to determine a compensated pressure level ($P_{npl}$) of said measured acoustic emission ($P_{spl}$).

The determination of the compensated pressure level may be based on characterizing the middle ear of the test subject as a Norton-equivalent circuit.

Thereby, an improved and more accurate compensation of pressure level of measured acoustic emissions from the ear of a test subject is provided.

Determination of the compensated pressure level ($P_{npl}$) may be based on compensating for the test subject's ear canal acoustics. For example, a test subject's ear canal acoustics may vary depending on a change of the ear canal shape over time, or on the position and orientation of the ear probe. Further, the emitted pressure primarily seeks to compensate for standing-wave resonances arising longitudinally along the ear canal. Because the emitted pressure or compensated pressure assume a uniform ear canal, this may also be a source of variation. Determination of the compensated pressure level ($P_{npl}$) may be based on compensating for the effect of the middle ear of the test subject.

Determination of the compensated pressure level ($P_{npl}$) may be based on compensating for the ear probe insertion position. For example, monitoring OAE response deterioration over time requires reproducibility in measuring OAE responses. However, it may be difficult to achieve identical ear-probe insertions between measurement sessions.

Determination of the compensated pressure level ($P_{npl}$) may be based on said measured acoustic emission ($P_{spl}$), a reflectance of the ear canal ($R_{ec}$), a source reflectance of the ear probe ($R_s$), and a one-way propagation coefficient ($T$) of the ear canal.

The term characterizing the middle ear of the test subject as a Norton-equivalent circuit comprises determining a compensated pressure level in dependence of a factor, $(T^2 + R_{ec})^{-1}$.

An advantage of the emitted pressure level $P_{epl}$ is that its magnitude is independent of the estimated ear-canal length constituted in the variable $T$. On the other hand, the Thévenin pressure level $P_{tpl}$ is extremely sensitive to errors in the ear-canal length. This is due to the factor $T^2 - R_{ec}$ in its denominator because identical lengths are encoded into $T^2$ and $R_{ec}$. Conversely, although the Norton pressure level is affected by length errors, it is to a much lesser degree because the factor in the denominator reads $T^2 + R_{ec}$. Furthermore, the Norton pressure level $P_{npl}$ phase is less sensitive to small length errors than the emitted pressure level $P_{epl}$. It is foreseen that the variable T may be calculated by using the ear-canal length, but may also be calculated in a different way.

The device may further be configured to determine the reflectance of the ear canal ($R_{ec}$) by:

- emitting at least one calibration stimulus, generated by the stimulus generator, into the ear canal of the test subject via said output unit,
- measuring an acoustic calibration response from the ear canal, via the input unit, and
- determining said reflectance of the ear canal ($R_{ec}$) based on said measured acoustic calibration response by the analysis unit.

The device may further be configured to determine the one-way propagation coefficient (7) of the ear canal by:

- emitting at least one calibration stimulus, generated by the stimulus generator, into the ear canal of the test subject via said output unit,
- measuring an acoustic calibration response from the ear canal, via the input unit, and
- determining said one-way propagation coefficient (7) of the ear canal based on said measured acoustic calibration response by the analysis unit.

Other means of determining said one-way propagation coefficient (7) of the ear canal is foreseen.

The device may further be configured to determine the source reflectance of the ear probe ($R_s$) from a preliminary Thévenin calibration.

The device may further be configured to determine said reflectance of the ear canal ($R_{ec}$) and said one-way propagation coefficient (7) of the ear canal by:

- emitting at least one calibration stimulus, generated by the stimulus generator, into the ear canal of the test subject, via said output unit,
- measuring an acoustic calibration response from the ear canal, via the input unit, and
- determining said reflectance of the ear canal ($R_{ec}$) and said one-way propagation coefficient (7) of the ear canal, based on said measured acoustic calibration response, by the analysis unit.

The calibration stimulus may refer to an audio stimulus that is of a type and is provided at a sound pressure level sufficient for primarily being reflected by the ear canal (i.e., the tympanic membrane and the ear canal walls). Thereby, the reflectance $R_{ec}$ may be measured based on the calibration stimulus emitted by the ear probe. The calibration stimulus may be of the type of a click or a chirp, but could also be a series of pure tones. The click or chirp calibration stimulus may e.g. have a duration measured in 10s of milliseconds, but longer or shorter duration is also foreseen.

The at least one acoustic stimulus, on the other hand, may refer to an audio stimulus that is of a type and is provided at a sound pressure level sufficient for primarily evoking OAEs in the cochlear of the test subject. Thereby, OAEs may be measured based on the acoustic stimulus, and the type of measured response can be designed based on the type and size of the stimulus.

Alternatively, or additionally, the device may further be configured to determine the measured impedance $Z_{ec}$ or admittance $Y_{ec}$ of the ear canal, the ear-canal characteristic impedance $Z_0$ or admittance $Y_0$, and the source impedance $Z_s$ or admittance $Y_s$ (for determining the various quantities below). Using reflectances just results in simpler equations.

The middle ear behaves more like a flow source than a pressure source. It is therefore reasonable to characterize it as a Norton-equivalent circuit. The equivalent flow source with the Norton flow level $U_{nfl}$ may now replace the pressure source with the Thévenin pressure level $P_{tpl}$. Thereby, the Norton flow level may be characterised by:

$$U_{nfl} = P_{epl} \frac{2}{Z_0(1 + R_{ec}/T^2)} = P_{spl} \frac{2T(1 - R_{ec}R_s)}{Z_0(1 + R_s)(T^2 + R_{ec})},$$

where $Z_0$ is the characteristic impedance of the ear canal.

Reporting OAE responses as acoustic flow may seem less intuitive by many, and thus the Norton flow level $\underline{U}_{nfl}$ may conveniently be converted into the Norton pressure level $P_{npl}$ by:

$$P_{npl} = U_{nfl}Z_0 = P_{spl} \frac{2T(1 - R_{ec}R_s)}{(1 + R_s)(T^2 + R_{ec})}.$$

The compensated pressure level ($P_{npl}$) of said measured acoustic emission may represent the sound pressure that would be emitted into an anechoic ear canal were the tympanic membrane is an ideal flow source, that is, were its impedance is infinitely large. The Thévenin pressure level $P_{tpl}$ on the other hand, represents an arbitrary equivalent pressure source behind the tympanic membrane.

The determination of the compensated pressure level ($P_{npl}$) may comprise calculating said compensated pressure level ($P_{npl}$) by the equation:

$$P_{npl} = P_{spl} \frac{2T(1 - R_{ec}R_s)}{(1 + R_s)(T^2 + R_{ec})}$$

The compensated pressure level ($P_{npl}$) may be calculated at a plurality of frequencies.

For example, a plurality of frequencies may comprise a plurality of frequencies above approximately 100 Hz, and/or a plurality of frequencies up to at least 20 kHz.

Thereby, the health of the cochlea may be assessed via the compensated pressure level determined at more than one frequency.

Further, therefore, the Norton pressure level $P_{npl}$ is independent of the input impedance of the middle ear, unlike the emitted pressure level $P_{epl}$ which does depend on the impedance of the middle ear. Furthermore, $P_{npl}$ and $P_{epl}$ are comparable in magnitude and phase when the impedance of the middle ear is large, whereas $P_{tpl}$ is significantly larger.

Use

In an aspect, use of a device as described above, and in the claims, is moreover provided.

A method:

In an aspect, a method of measuring OAEs of a test subject is furthermore provided by the present application.
The method may comprise generating at least one acoustic stimulus.
The at least one acoustic stimulus may be generated by a stimulus generator.
The method may comprise emitting the at least one acoustic stimulus into the ear canal of the test subject.
The at least one acoustic stimulus may be emitted by an output unit of an ear probe.
The method may comprise measuring an acoustic emission ($P_{spl}$) from the ear of the test subject.
The acoustic emission ($P_{spl}$) may be measured by an input unit of the ear probe.
The method may comprise receiving said measured acoustic emission ($P_{spl}$).
The measured acoustic emission ($P_{spl}$), and/or a signal representing said measured acoustic emission ($P_{spl}$), may be received by an analysis unit from the input unit of the ear probe. Accordingly, the analysis unit may be in communication (wirelessly or wired) with the input unit.
The method may comprise determining a compensated pressure level ($P_{npl}$) of said measured acoustic emission ($P_{spl}$).
The compensated pressure level ($P_{npl}$) may be determined by the analysis unit.
The determination of the compensated pressure level ($P_{npl}$) may be based on characterizing the middle ear of the test subject as a Norton-equivalent circuit.
The step of characterizing the middle ear of the test subject as a Norton-equivalent circuit may comprise determining a compensated pressure level in dependence of a factor $(T^2 + R_{ec})^{-1}$, where $T$ is a one-way propagation coefficient of the ear canal and $R_{ec}$ is a reflectance of the ear canal.
The method may further comprise determining the reflectance of the ear canal ($R_{ec}$) by:

- emitting at least one calibration stimulus, generated by the stimulus generator, into the ear canal of the test subject via said output unit,
- measuring an acoustic calibration response from the ear canal, via the input unit, and
- determining said reflectance of the ear canal ($R_{ec}$) based on said measured acoustic calibration response by the analysis unit.

The method may further comprise determining the one-way propagation coefficient (7) of the ear canal by:

- emitting at least one calibration stimulus, generated by the stimulus generator, into the ear canal of the test subject via said output unit,
- measuring an acoustic calibration response from the ear canal, via the input unit, and
- determining said one-way propagation coefficient (7) of the ear canal based on said measured acoustic calibration response by the analysis unit.

Other means of determining said one-way propagation coefficient (7) of the ear canal is foreseen.
The method may further comprise determining the source reflectance of the ear probe ($R_s$) from a preliminary Thévenin calibration.
The method may further comprise determining the reflectance of the ear canal ($R_{ec}$) and the one-way propagation coefficient (7) of the ear canal by:

- emitting at least one calibration stimulus, generated by the stimulus generator, into the ear canal of the test subject via said output unit,
- measuring an acoustic calibration response from the ear canal, via the input unit, and
- determining said reflectance of the ear canal ($R_{ec}$) and said one-way propagation coefficient (7) of the ear canal, based on said measured acoustic calibration response by the analysis unit.

The step of determining the compensated pressure level ($P_{npl}$) may comprise calculating said compensated pressure level ($P_{npl}$) by the equation:

$$P_{npl} = P_{spl} \frac{2T(1 - R_{ec}R_s)}{(1 + R_s)(T^2 + R_{ec})}$$

The compensated pressure level ($P_{npl}$) may be calculated at a plurality of frequencies.

It is intended that some or all of the structural features of the device described above, or in the claims can be combined with embodiments of the method, when appropriately substituted by a corresponding process and vice versa. Embodiments of the method have the same advantages as the corresponding devices.

A computer readable medium or data carrier

In an aspect, a tangible computer-readable medium (a data carrier) storing a computer program comprising program-code means (instructions) for instructing (causing) a data processing system (a computer) to perform (carry out) at least some (such as a majority or all) of the (steps of the) method described above, and in the claims, when said computer program is executed on the data processing system is furthermore provided by the present application.

A computer program

In an aspect, a computer program is provided. The computer program (product) may comprise instructions which, when the program is executed by a device (a computer), cause the device (computer) to carry out (steps of) the method described above, and in the claims.

A data processing system

In an aspect, a data processing system comprising a processor and program-code means for instructing (causing) the processor to perform at least some (such as a majority or all) of the steps of the method described above, and in the claims is furthermore provided by the present application.

A system

In a further aspect, a system comprising a device (as described above and in the claims) and an auxiliary device, is moreover provided.

The system may be adapted to establish a communication link between the device and the auxiliary device to provide that information/data can be exchanged or forwarded from one to the other.

An APP

In a further aspect, a non-transitory application, termed an APP, is furthermore provided by the present disclosure. The APP comprises executable instructions configured to be executed on an auxiliary device, e.g., forming part of said device or being another portable (tablet) device in communication with said device, to implement a user interface for a device or system as described above, and in the claims.

BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features, and/or technical effect will be apparent from and elucidated with reference to the illustrations described hereinafter in which:

FIG. 1 shows an exemplary device according to the present application.

FIG. 2 shows the possible varying insertion locations and positions of an ear probe.

FIGS. 3A and 3B show measurements of the reflectance and of OAEs, respectively.

FIG. 4 shows exemplary relative OAE response magnitudes and phases in an occluded-ear simulator.

The figures are schematic and simplified for clarity, and they just show details which are essential to the understanding of the disclosure, while other details are left out. Throughout, the same reference signs are used for identical or corresponding parts.

Further scope of applicability of the present disclosure will become apparent from the detailed description given hereinafter. However, it should be understood that the detailed description and specific examples, while indicating preferred embodiments of the disclosure, are given by way of illustration only. Other embodiments may become apparent to those skilled in the art from the following detailed description.

DETAILED DESCRIPTION OF EMBODIMENTS

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the device and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

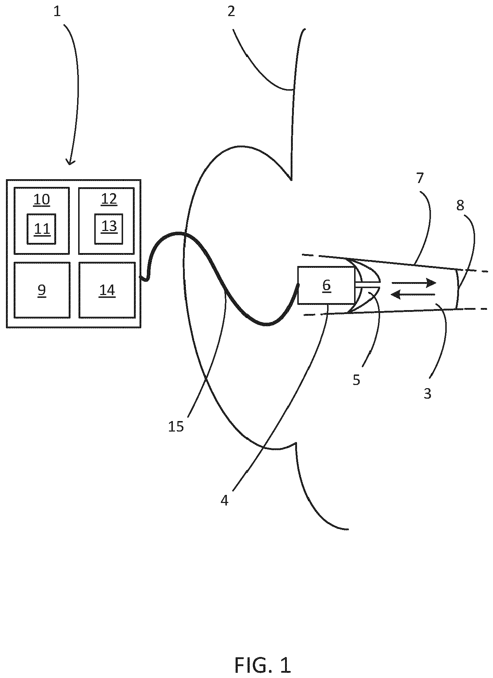

FIG. 1 shows an exemplary device according to the present application.

In FIG. 1, the device 1 is shown to be located at and/or in connection with the head 2 of a test subject.

The device 1 may be configured to assess the health of the cochlea of a test subject. For example, the device 1 may be suitable for measuring otoacoustic emissions (OAE) of a test subject.

The device 1 is shown to comprise two elements, but may of course be combined to only one element, or alternatively be divided into several elements.

In FIG. 1, a first element of the device 1 may be arranged inside the ear canal 3 of the test subject. Alternatively, or additionally, the first element may be arranged at least partly outside the ear canal 3. The first element is shown to be an ear probe 4 suitable for insertion into the ear canal 3.

The ear probe 4 may comprise an ear tip 5, which may be releasably attached to an ear-probe body 6. When the ear probe 4 has been inserted correctly into the ear canal 3, the ear tip 5 may provide a barometric seal toward the ear-canal walls 7 of the ear canal 3. The device 1 may (via the ear probe 4) provide at least one stimulus (e.g., an acoustic stimulus or a calibration stimulus) into the ear canal 3 of the test subject 2 in a direction towards the eardrum 8 and receive a reflected part of said stimulus and/or an otoacoustic emission, as indicated by the two arrows in the ear canal 3.

A second element of the device 1 is shown to be arranged outside the ear canal 3 of the test subject 2. Alternatively, or additionally, the second part (or at least some elements of the second part) could be combined with the ear probe 4.

The device 1 may comprise a stimulus generator 9. The stimulus generator 9 may be configured to generate at least one acoustic stimulus. Said acoustic stimulus may be introduced/emitted into the ear canal 3 of the test subject 2 via an output unit 10 (e.g., comprising a speaker 11) of the ear probe 4.

The ear probe 4 may comprise an input unit 12 (e.g., comprising a microphone 13). The input unit 12 may be configured to measure an acoustic emission ($P_{spl}$) from the ear of the test subject 2, and may be configured to provide an electrical input signal in dependence of the acoustic emission.

The device 1 may comprise an analysis unit 14. The analysis unit 14 may be connected to the ear probe 4, and may be configured to receive the measured acoustic emission ($P_{spl}$) from said input unit 12. The analysis unit 14 may be configured to determine a compensated pressure level ($P_{npl}$) of said measured acoustic emission ($P_{spl}$), where the determination of the compensated pressure level is based on characterizing the middle ear of the test subject 2 as a Norton-equivalent circuit.

As indicated in FIG. 1, the second element of the device 1 may be connected to the first element. The first and second elements may be connected by a wired or a wireless connection 15, or may alternatively form one unit.

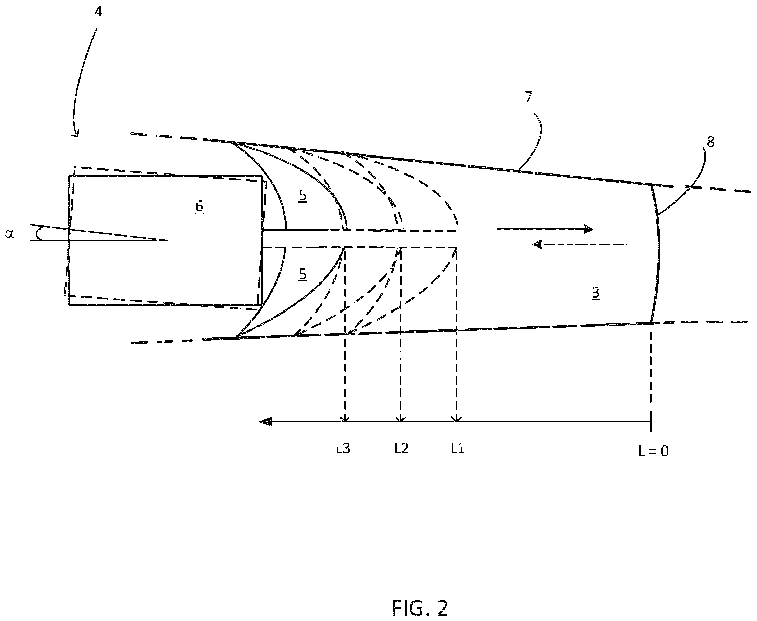

FIG. 2 shows the possible varying insertion locations and positions of an ear probe.

Determination of a compensated pressure level ($P_{npl}$) may be based on compensating for the test subject's ear-canal acoustics. For example, a test subject's ear-canal acoustics may vary depending on the position and orientation of the ear probe 4. For example, monitoring for ototoxic drugs may cause OAE responses to deteriorate over time for which reason such monitoring process requires that an OAE response can be accurately reproduced.

Further, standing waves between the tympanic membrane 8, where an OAE enters the ear canal 3, and the ear probe 4 may influence the OAE response, resulting in amplification of the OAE response near resonance frequencies. The result is that OAE responses may vary significantly when the ear probe 4 is not placed in the exact same location between measurement sessions.

In FIG. 2, it is illustrated that the ear probe 4 may unintentionally be inserted at different distances from the tymphanic membrane 8. This is illustrated by the distances L1, L2, and L3, which makes reproducibility of the OAE test difficult.

Also, as illustrated in FIG. 2, the ear probe 4 (and in particular the ear-probe body 6) may be positioned at a varying angle $\alpha$ from test to test, which makes reproducibility of the OAE test difficult.

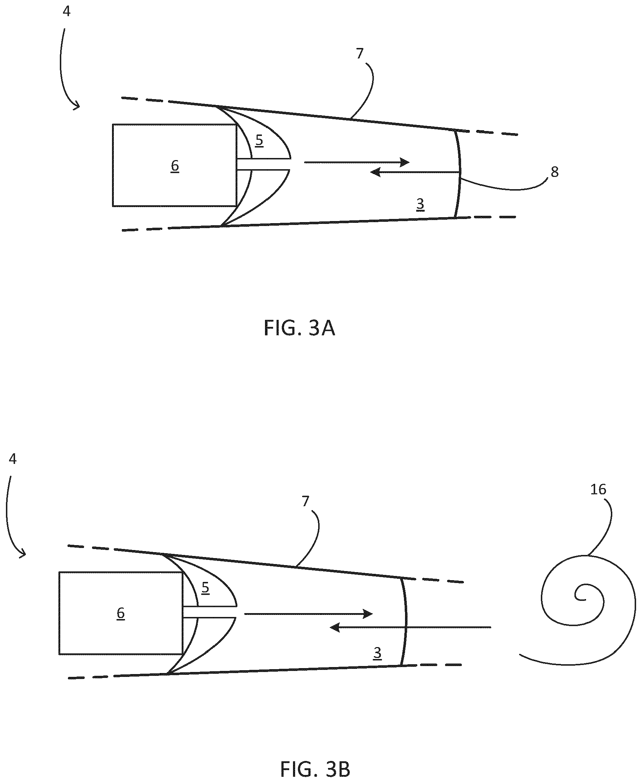

FIGS. 3A and 3B show measurements of the reflectance and of OAEs, respectively.

In FIG. 3A, a calibration stimulus may be emitted into the ear canal 3 of the test subject. The type and sound pressure level of said calibration stimulus may be sufficient to primarily being reflected by the ear canal 3 (i.e., the tympanic membrane 8 and the ear canal walls 7). Thereby, the reflectance $R_{ec}$ can be measured based on the calibration stimulus emitted by the ear probe 4.

In FIG. 3B, at least one acoustic stimulus may be emitted into the ear canal 3 of the test subject. The type and sound-pressure level of said acoustic stimulus may be sufficient for primarily evoking OAEs in the cochlear 16 of the test subject. Thereby, OAEs may be measured based on said acoustic stimulus.

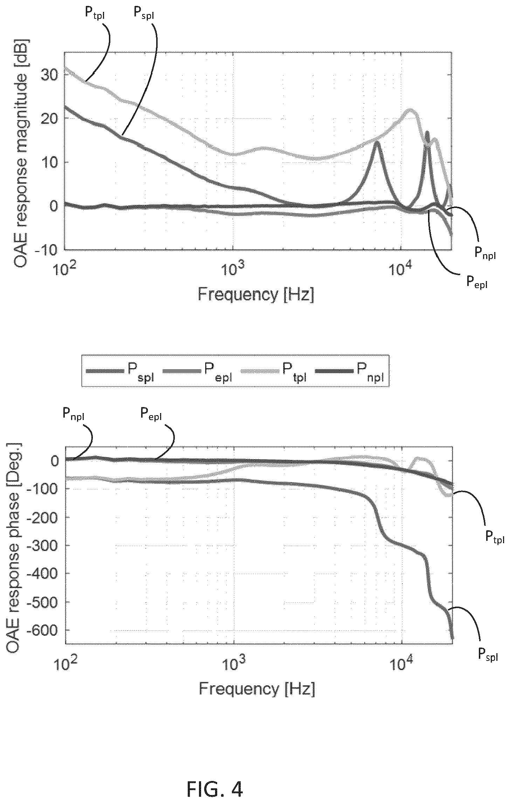

FIG. 4 shows exemplary relative OAE response magnitudes and phases in an occluded-ear simulator.

To illustrate the merits of the Norton pressure level, the simulated OAE response in an occluded-ear simulator was measured (similar to [2]). That is, a reference microphone at the coupler termination (representing the tympanic membrane) is operated as an electrostatic speaker. The reference coupler microphone can now be approximated as an ideal displacement source which, when scaled by $1/(j\omega))$, acts as an ideal flow source, i.e., the Norton flow level $U_{nfl}$. The ear probe was calibrated according to [4] [5] and the ear-canal reflectance measured according to [6].

FIG. 4 shows the relative OAE response magnitudes and phases, i.e., scaled by $1/(U_{nfl} Z_0)$. [3] provides a detailed investigation of the impact of insertion depth and ear-probe insertion angle on the ear-canal pressure level $P_{spl}$ and emitted pressure level $P_{epl}$.

It is illustrated how the measured OAE response $P_{spl}$ (blue curve) without any compensation is amplified by the compliance of the ear canal at low frequencies and by standing waves at 7 and 14 kHz. When the insertion of the ear probe varies, these resonances are translated in frequency and the OAE response changes.

The emitted pressure level $P_{epl}$ (red curve) is unaffected by the ear-canal acoustics, but remains effected by the simulated middle-ear impedance in the occluded-ear simulator. This is evident from the 2 dB decrease in relative OAE response at 1 and 3 kHz.

The Thévenin pressure level $P_{tpl}$ (yellow curve) is unaffected by the middle-ear impedance per se, but varies significantly with minor measurement errors and variations in the ear-canal propagation coefficient $T$.

The Norton pressure level $P_{npl}$ is largely comparable to the emitted pressure level $P_{epl}$ in the occluded-ear simulator, but is unaffected by the middle-ear resonances at 1 and 3 kHz. In real ears, where one might encounter middle-ear impedances with lower magnitudes, the difference between $P_{epl}$ and $P_{npl}$ may by larger.

An advantage of the emitted pressure level $P_{epl}$ is that its magnitude is independent of the estimated ear-canal length constituted in the variable $T$. On the other hand, the Thévenin pressure level $P_{tpl}$ is extremely sensitive to errors in ear-canal propagation coefficient. This is due to the factor $T^2 - R_{ec}$ in its denominator because identical delays, resulting primarily from the ear-canal length, are encoded into $T^2$ and $R_{ec}$. Conversely, although the Norton pressure level is affected by errors in the ear-canal propagation coefficient, it is to a much lesser degree because the factor in the denominator reads $T^2 + R_{ec}$. Furthermore, the Norton pressure level $P_{npl}$ phase appears to be less sensitive to small length errors than the emitted pressure level $P_{epl}$ phase.

It is intended that the structural features of the devices described above, either in the detailed description and/or in the claims, may be combined with steps of the method, when appropriately substituted by a corresponding process.

As used, the singular forms "a," "an," and "the" are intended to include the plural forms as well (i.e., to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element, but an intervening element may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method are not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects.

The claims are not intended to be limited to the aspects shown herein but are to be accorded the full scope consistent with the language of the claims, wherein reference to an element in the singular is not intended to mean "one and only one" unless specifically so stated, but rather "one or more". Unless specifically stated otherwise, the term "some" refers to one or more.

REFERENCES

[1] Charaziak, K. K., & Shera, C. A. (2017). Compensating for ear-canal acoustics when measuring otoacoustic emissions. J. Acoust. Soc. Am. 141(1), 515-531.

[2] Allen, J. B. (1986). Measurement of eardrum acoustic impedance. In J. Allen, J. Hall, A. Hubbard, S. Neely, & A. Tubis (Eds.), Peripheral Auditory Mechanisms (pp. 44-51). Springer-Verlag.

[3] Nørgaard, K. R., Charaziak, K. K., & Shera, C. A. (2019). A comparison of ear-canal-reflectance measurement methods in an ear simulator. J. Acoust. Soc. Am. 146(2), 1350-1361.

[4] Nørgaard, K. R., Fernandez-Grande, E., & Laugesen, S. (2017). Incorporating evanescent modes and flow losses into reference impedances in acoustic Thévenin calibration. J. Acoust. Soc. Am. 142(5), 3013-3024.

[5] Nørgaard, K. R., Fernandez-Grande, E., & Laugesen, S. (2018). A coupler-based calibration method for ear-probe microphones. J. Acoust. Soc. Am. 144(4), 2294-2299.

[6] Nørgaard, K. R., Fernandez-Grande, E., & Laugesen, S. (2019). Compensating for oblique ear-probe insertions in ear-canal reflectance measurements. J. Acoust. Soc. Am. 145(6), 3499-3509.

**Claims**

1. Device for measuring otoacoustic emissions (OAE) of a test subject, the device comprising:

   - a stimulus generator configured to generate at least one acoustic stimulus,
   - an ear probe connected to said stimulus generator, where the ear probe comprises an output unit for emitting said at least one acoustic stimulus into an ear canal of the test subject, and an input unit for measuring an acoustic emission ($P_{spl}$) from the ear of the test subject, and
   - an analysis unit connected to the ear probe, where the analysis unit is configured to receive said measured acoustic emission ($P_{spl}$), and to determine a compensated pressure level ($P_{npl}$) of said measured acoustic emission ($P_{spl}$), where the middle ear of the test subject is characterized as a Norton-equivalent circuit in the determination of the compensated pressure level ($P_{npl}$).

2. Device according to claim 1, wherein determination of the compensated pressure level ($P_{npl}$) is based on compensating for the test subject's ear canal acoustics, the effect of the middle ear of the test subject, and/or for the ear probe insertion position on the acoustic emission ($P_{spl}$).

3. Device according to any one of the preceding claims, wherein determination of the compensated pressure level ($P_{npl}$) is based on said measured acoustic emission ($P_{spl}$), a reflectance of the ear canal ($R_{ec}$), a source reflectance of the ear probe ($R_s$), and a one-way propagation coefficient (7) of the ear canal.

4. Device according to any one of the preceding claims, wherein the term, characterizing the middle ear of the test subject as a Norton-equivalent circuit, comprises determining a compensated pressure level in dependence of a factor, $(T^2 + R_{ec})^{-1}$.

5. Device according to any one of claims 3-4, wherein the device is further configured to determine said reflectance of the ear canal ($R_{ec}$) and/or said one-way propagation coefficient (7) of the ear canal by:

   - emitting at least one calibration stimulus, generated by the stimulus generator, into the ear canal of the test subject, via said output unit,
   - measuring an acoustic calibration response from the ear canal, via the input unit, and

   - determining said reflectance of the ear canal ($R_{ec}$) and/or said one-way propagation coefficient (7) of the ear canal, based on said measured acoustic calibration response, by the analysis unit.

6. Device according to any one of the preceding claims, wherein the compensated pressure level ($P_{npl}$) of said measured acoustic emission represents the sound pressure that would be emitted into an anechoic ear canal were the tympanic membrane is an ideal flow source.

7. Device according to any one of the preceding claims, wherein determination of the compensated pressure level ($P_{npl}$) comprises calculating, at a plurality of frequencies, said compensated pressure level ($P_{npl}$) by the equation:

$$P_{npl} = P_{spl} \frac{2T(1 - R_{ec}R_s)}{(1 + R_s)(T^2 + R_{ec})}$$

8. Method of measuring otoacoustic emissions (OAE) of a test subject, the method comprising:

   - generating at least one acoustic stimulus, by a stimulus generator,
   - emitting the at least one acoustic stimulus into the ear canal of the test subject, by an output unit of an ear probe,
   - measuring an acoustic emission ($P_{spl}$) from the ear of the test subject, by an input unit of the ear probe,
   - receiving said measured acoustic emission ($P_{spl}$), by an analysis unit, and
   - determining a compensated pressure level ($P_{npl}$) of said measured acoustic emission ($P_{spl}$), by the analysis unit, where the middle ear of the test subject is characterized as a Norton-equivalent circuit in the determination of the compensated pressure level ($P_{npl}$).

9. Method according to claim 8, wherein characterizing the middle ear of the test subject as a Norton-equivalent circuit comprises determining a compensated pressure level in dependence of a factor ($T^2 + R_{ec}$)$^{-1}$, where $T$ is a one-way propagation coefficient of the ear canal and $R_{ec}$ is a reflectance of the ear canal.

10. Method according to any one of claims 8-9, wherein the method further comprises determining the reflectance of the ear canal ($R_{ec}$) and/or the one-way propagation coefficient (7) of the ear canal by:

   - emitting at least one calibration stimulus, generated by the stimulus generator, into the ear canal of the test subject, via said output unit,
   - easuring an acoustic calibration response from the ear canal, via the input unit, and
   - determining said reflectance of the ear canal ($R_{ec}$) and/or said one-way propagation coefficient (7) of the ear canal, based on said measured acoustic calibration response, by the analysis unit.

11. Method according to any one of claims 8-10, wherein determination of the compensated pressure level ($P_{npl}$) comprises calculating, at a plurality of frequencies, said compensated pressure level ($P_{npl}$) by the equation:

$$P_{npl} = P_{spl} \frac{2T(1 - R_{ec}R_s)}{(1 + R_s)(T^2 + R_{ec})}$$

12. A computer program comprising instructions which, when the program is executed by a device, cause the device to carry out the steps of the method according to any one of claims 8-11.

FIG. 1

FIG. 2

FIG. 3A

FIG. 3B

FIG. 4

Europäisches
Patentamt

European
Patent Office

Office européen
des brevets

# EUROPEAN SEARCH REPORT

**Application Number**

EP 24 15 3141

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/257188 A1 (FATHER FLANAGANS BOYS HOME DOING BUSINESS AS BOYS TOWN NATIONAL RES HO) 24 December 2020 (2020-12-24) <br> * page 3, line 14 - page 4, line 27; figure 1 * <br> * page 12, line 23 - page 14, line 14 * <br> * page 33, lines 16-23 * | 1-6, 8-10,12 | INV. <br> A61B5/12 |
| X | US 5 792 072 A (KEEFE DOUGLAS H [US]) 11 August 1998 (1998-08-11) <br> * column 9, line 4 - column 10, line 35; figure 1 * | 1-6, 8-10,12 | |
| A,D | CHARAZIAK KAROLINA K ET AL: "Compensating for ear-canal acoustics when measuring otoacoustic emissions", <br> THE JOURNAL OF THE ACOUSTICAL SOCIETY OF AMERICA, AMERICAN INSTITUTE OF PHYSICS, 2 HUNTINGTON QUADRANGLE, MELVILLE, NY 11747, vol. 141, no. 1, <br> 25 January 2017 (2017-01-25), pages 515-531, XP012215647, <br> ISSN: 0001-4966, DOI: 10.1121/1.4973618 [retrieved on 2017-01-25] <br> * abstract * <br> * IV DISCUSSION: E. Alternative methods for removing ear-canal acoustics from OAEs, p. 525-526 * | 1-12 | |

TECHNICAL FIELDS
SEARCHED (IPC)

A61B
H04R

-/--

The present search report has been drawn up for all claims

| Place of search | Date of completion of the search | Examiner |
|---|---|---|
| Berlin | 3 June 2024 | Trachterna, Morten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

**Application Number**

EP 24 15 3141

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | N?RGAARD KREN MONRAD ET AL: "A systematic study on effects of calibration-waveguide geometry and least-squares formulation on ear-probe source calibrations", THE JOURNAL OF THE ACOUSTICAL SOCIETY OF AMERICA, AMERICAN INSTITUTE OF PHYSICS, 2 HUNTINGTON QUADRANGLE, MELVILLE, NY 11747, vol. 151, no. 1, 31 January 2022 (2022-01-31), pages 634-649, XP012263128, ISSN: 0001-4966, DOI: 10.1121/10.0009325 [retrieved on 2022-01-31] * abstract * * II. Background, p. 635-636. * ----- | 1-12 | |
| | | | **TECHNICAL FIELDS SEARCHED (IPC)** |

The present search report has been drawn up for all claims

| Place of search | Date of completion of the search | Examiner |
|---|---|---|
| Berlin | 3 June 2024 | Trachterna, Morten |

EPO FORM 1503 03.82 (P04C01)

## ANNEX TO THE EUROPEAN SEARCH REPORT
## ON EUROPEAN PATENT APPLICATION NO.

EP 24 15 3141

This annex lists the patent family members relating to the patent documents cited in the above-mentioned European search report.
The members are as contained in the European Patent Office EDP file on
The European Patent Office is in no way liable for these particulars which are merely given for the purpose of information.

03-06-2024

| Patent document cited in search report | | Publication date | Patent family member(s) | | Publication date |
|---|---|---|---|---|---|
| WO 2020257188 | A1 | 24-12-2020 | NONE | | |
| US 5792072 | A | 11-08-1998 | CA | 2239583 A1 | 12-06-1997 |
| | | | EP | 0957764 A2 | 24-11-1999 |
| | | | EP | 1415592 A1 | 06-05-2004 |
| | | | US | 5792072 A | 11-08-1998 |
| | | | WO | 9720501 A2 | 12-06-1997 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CHARAZIAK, K. K. ; SHERA, C. A.** Compensating for ear-canal acoustics when measuring otoacoustic emissions. *J. Acoust. Soc. Am.,* 2017, vol. 141 (1), 515-531
- Measurement of eardrum acoustic impedance. **ALLEN, J. B.** Peripheral Auditory Mechanisms. Springer-Verlag, 1986, 44-51
- **NØRGAARD, K. R ; CHARAZIAK, K. K. ; SHERA, C. A.** A comparison of ear-canal-reflectance measurement methods in an ear simulator. *J. Acoust. Soc. Am.,* 2019, vol. 146 (2), 1350-1361

- **NØRGAARD, K. R ; FERNANDEZ-GRANDE, E. ; LAUGESEN, S.** Incorporating evanescent modes and flow losses into reference impedances in acoustic Thévenin calibration. *J. Acoust. Soc. Am.,* 2017, vol. 142 (5), 3013-3024
- **NØRGAARD, K. R. ; FERNANDEZ-GRANDE, E. ; LAUGESEN, S.** A coupler-based calibration method for ear-probe microphones. *J. Acoust. Soc. Am.,* 2018, vol. 144 (4), 2294-2299
- **NØRGAARD, K. R. ; FERNANDEZ-GRANDE, E. ; LAUGESEN, S.** Compensating for oblique ear-probe insertions in ear-canal reflectance measurements. *J. Acoust. Soc. Am.,* 2019, vol. 145 (6), 3499-3509